# EUROPEAN PATENT SPECIFICATION

(11) **EP 3 349 594 B1**
(45) Date of publication and mention of the grant of the patent: **22.04.2020**
(21) Application number: 15781394.0
(22) Date of filing: 14.09.2015
(51) Int. Cl.: A23L 27/40, A23L 33/165, A61P 9/12

(54) **SALT SUBSTITUTE COMPOSITION AND USE THEREOF**
ERSATZPRODUKT FÜR SALZ, UND DESSEN VERWENDUNG
SUBSTITUT DE SEL ET SON UTILISATION

(43) Date of publication of application: 25.07.2018
(73) Proprietor: PODRAVKA d.d., 48000 Koprivnica (HR)
(72) Inventor: CEPANEC, Ivica, 10314 Kriz (HR); RANILOVIC Jasmina, 48000 Koprivnica (HR); CVETKOVIC, Tanja, 48000 Koprivnica (HR); VUGRINEC, Sasenka, 48000 Koprivnica (HR)
(74) Representative: Vukmir, Mladen
(86) International application number: PCT/HR2015/000016
(87) International publication number: WO 2017/046620

(56) References cited:
- EP-A1- 0 417 062
- EP-A1- 0 766 927
- BE-A1- 902 690
- FR-A1- 2 773 955
- US-A- 4 985 593
- US-A1- 2005 197 402
- US-A1- 2009 104 330

## Description

### Technical Field

The invention relates to a new salt substitute composition based on sodium chloride (NaCl), potassium chloride (KCl), and potassium magnesium citrate [K₄Mg(C₆H₅O₇)₂], its preparation and use.

### Technical Problem

Technical problem is related to more efficient salt substitute providing effective masking of bitter, acrid and/or metallic taste, clean saltiness perception and saltiness intensity of potassium chloride-containing salt substitutes.

The composition of the invention provides:
(i) effective masking of potassium chloride-derived bitterness and other offensive taste;
(ii) clean saltiness with a good perception intensity; and
(iii) blood pressure lowering action, in the same time.

### Previous State of the Art

Salt substitutes are important food products that are used as ordinary salt (NaCl) replacements in order to reduce sodium intake. The latter was recognized as important health risk factor of modern western people diet. High intake of sodium can lead to development of various cardiovascular diseases among which the most important one is essential hypertension; see literature reference 1:
1) M. P. Blaustein, F. H. Leenen, L. Chen, V. A. Golovina, J. M. Hamlin, T. L. Pallone, J. W. Van Huysse, J. Zhang, W. G. Wier: How NaCl raises blood pressure: a new paradigm for the pathogenesis of salt-dependent hypertension, Am. J. Physiol. Heart Circ. Physiol. 302 (2012) H1031-49.
   The major group of salt substitutes are based on salty taste of potassium chloride (KCl). But, beside salty, KCl is characterized by offensive bitter and somewhat acrid and/or metallic tastes. In this manner an unsolved technical problem is how to achieve efficient masking of potassium chloride-based salt substitute off-flavours.
   Beside masking of bitterness and other off-flavours of potassium chloride the important characteristic is to keep adequate saltiness of salt substitutes since KCl is of significantly lower saltiness than ordinary salt (NaCl) . In this manner the same weight of potassium chloride in one typical meal is much less salty than the same amount of ordinary salt. Thus an ideal taste-improving agent for potassium-chloride based salt substitutes would have two characteristics:
   (i) good bitterness- and off-flavours-masking; and
   (ii) saltiness enhancing activities.

   GB1160191A discloses a salt substitute composition based on mixtures of sodium chloride (NaCl) and potassium chloride (KCl). There is generically mentioned that various compounds can be used for masking bitterness of potassium chloride: e.g. "potassium and calcium formates, magnesium citrate, dipotassium succinate, various citrates, tartrates, gluconates, ascorbates, cyclamates, glutamates, and ionexchange resins", but without describing exact procedure how to achieve this; see literature reference 2:
2) GB1160191A, Culinary seasoning salt composition and method for making the same; Morton Int. Inc. (US).
   The use of certain citrate salts as bad taste-masking agents in salt substitute formulations has been described.
   Thus Mallet discloses a low-salt food curing agent containing 35-55% NaCl, 25-45% KCl, 4.5-9% tripotassium citrate (K₃C₆H₅O₇), and 0.5-5% of magnesium carbonate, obviously as anti-caking agent; see literature reference 3:
3) WO2015024578A1, L. Mallet: Novel low-salt food curing agent; Euro Exp. Sarl (MC).
   EP0417062A1 teaches about the composition of salt substitute based on sodium chloride (NaCl), potassium chloride (KCl), and magnesium hydrogencitrate pentahydrate [MgH (C₆H₅O₇) •5H₂O], characterized by the weight ratios of NaCl:KCl = 5:1-1:1, KCl : MgH(C₆H₅O₇)•5H₂O = 5:1-1:5; with preferred composition of NaCl : KCl : MgH (C₆H₅O₇) •5H₂O = 2.6:1:0.08 ; see literature reference 4:
4) EP0417062A1, H. Schwaiger: Table salt with a reduced sodium content; Bad Ischler Salz (AT).
   US5562942A discloses a salt composition that contains 30-75% NaCl, 25-70% KCl, and 5-60% citrate salt selected from the group consisting of: tripotassium citrate (K₃C₆H₅O₇), trisodium citrate (Na₃C₆H₅O₇), and eventually trimagnesium citrate [Mg₃(C₆H₅O₇)₂], and tricalcium citrate [Ca₃(C₆H₅O₇)₂]. The use of tripotassium citrate is preferred, whilst the use of trimagnesium and tripotassium citrates are suggested only because of better balanced minerals intake; see literature reference 5:
5) US5562942A, H.-S. Koh, Y. Kawashima, T. Hasimoto: Salt compositions.
   The use of potassium citrate, in combination with potassium dihydrogenphosphate (KH₂PO₄) or calcium dihydrogenphosphate [Ca(H₂PO₄)₂] as taste-improving agents for salt substitutes based on potassium chloride (KCl) with or without sodium chloride (NaCl) was also described; see literature reference 6:
6) WO2009099466A1, K. R. Vadlamani, D. Friday, A. Broska, J. Miller: Methods and compositions for reducing sodium content in food products; Campbel Soup Co. (US).
   EP0766927A1 discloses a dietary salt mixture composition that contains 20-40% NaCl, 20-40% KCl, 10-20% potassium citrate, 0.6-1% magnesium citrate or magnesium glutamate, 10-15% dried vegetables and spices, 5-8% sodium glutamate, and optionally, 0.03-0.2% nutritionally acceptable antioxidant, 1-3% lubricant, preferably starch, and eventually other food ingredient like sugar and/or sweetener; see literature reference 7:
7) EP0766927A1, N. E. Gyarmathy, C. Farsang: Dietary salt mixture poor in sodium comprising also potassium, magnesium and a mixture of vegetables and spices.
   Although this document describes a combination of "potassium citrate" + "magnesium citrate" use, there is no precise information which citrates are used, presumably these were tripotassium citrate (K₃C₆H₅O₇) and trimagnesium citrate [Mg₃(C₆H₅O₇)₂]. Additionally, the person skilled in the art cannot elucidate any eventual synergistic action of "potassium citrate" and "magnesium citrate" to improve off-flavours in KCl-based composition due to:
   (i) a very complex mixture that contains a long list of various vegetable and spices;
   (ii) an optional use of "magnesium citrate", beside "magnesium glutamate"; and
   (iii) a parallel use of strong umami flavour enhancer sodium glutamate.

   According to our best knowledge it seems that the closest prior art is BE902690A1, which teaches about salt substitute based on potassium and magnesium salts of weak organic acids and/or phosphoric acid. As weak organic acids: tartaric, lactic, citric, gluconic, or acetic acid salts are used. As phosphates, hydrogenphosphates and dihydrogenphosphates are employed. One of preferred embodiment disclosed in Example 5 is of the following composition: 57% NaCl, 25% KCl, 8% tripotassium citrate (K₃C₆H₅O₇), 5% trimagnesium citrate [Mg₃(C₆H₅O₇)₂], and obviously ad 100% (= 5%) potassium or sodium hydrogenphosphate (M₂HPO₄; M= Na,K) or dihydrogenphosphate (MH₂PO₄; M= Na,K); see literature reference 8.
8) BE902690A1, L. Takanen: Compositions containing sodium, potassium and magnesium salts-including carboxylate (s) and/or phosphonate(s), as substitute for sodium chloride in foodstuff or as table salt; Cederroth Nordic AB (SE).

Although this document describes simultaneous use of tripotassium citrate (K₃C₆H₅O₇) and trimagnesium citrate [Mg₃(C₆H₅O₇)₂] in the salt substitute based on KCl + NaCl, this formulation includes also parallel use of acidic phosphoric acid salts, hydrogenphosphates and dihydrogenphosphates, for which it is known that they generate slightly acidic pH reaction what result in significant impact on taste perception of such salt substitutes.

According to our best knowledge, the use of specific potassium magnesium citrate of the chemical formula K₄Mg(C₆H₅O₇)₂, with stoichiometric ratio of:

### n(K) : n(Mg) : n(citrate) = 4:1:2

as a off-flavours masking and taste-improving agent in potassium chloride-based salt substitutes has not been described in related art.

This salt was disclosed by Walsdorf and Alexandrides as a new compound with use as dietary supplement for supplementation with potassium and magnesium; see literature reference 9:
9) EP0380829A1, N. B. Walsdorf, G. Alexandrides: Magnesium potassium citrate; Mission Pharma Co. (US).
   Among other uses of potassium magnesium citrate [K₄Mg(C₆H₅O₇)₂], Pak disclosed the use of this citrate salt as food supplement for treatment of essential hypertension; see literature reference 10:
10) US2008193525A1, C. Y. C. Pak: Potassium-magnesium citrate as a surrogate of the dash diet in managing hypertension; University Texas (US).

A commercial product called "Naresa" is known. It comprises sodium chloride, potassium chloride and magnesium-potassium-citrate, and provides 20,0 and 23,0 g/100 of sodium and potassium, respectively.

The present invention represents simple solution for resolving bad taste of potassium chloride-based salt substitutes and blood pressure lowering activity in the same time, as will be disclosed in detailed description of this invention.

### Summary of Invention

The present invention, which is defined by the claims, relates to the new composition of salt substitute comprising sodium chloride (NaCl), potassium chloride (KCl), and specific double salt potassium magnesium citrate (**1**) of formula K₄Mg(C₆H₅O₇)₂: 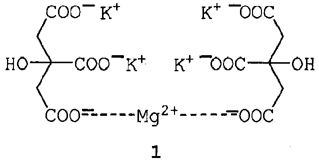 in the relative mass fractions defined later, which effectively masks bad offensive taste that originates from potassium chloride, enhances salty taste perception, and acts as the blood pressure lowering agent in the same time. The composition optionally contains one or more auxiliary food ingredient, e.g. anti-caking agent.

The composition is used as a salt substitute; a seasoning; in manufacturing of food products with reduced sodium content; or as adjuvant for prevention and treatment of diseases and conditions connected with high sodium consumption, e.g. hypertension; potassium and magnesium deficiencies treating agent; as well as dietetic salt for use in animals.

### Detailed Description

The present invention discloses the new specific composition of salt substitute comprising:
(i) sodium chloride,
(ii) potassium chloride,
(iii) potassium magnesium citrate (K₄Mg(C₆H₅O₇)₂; **1**) 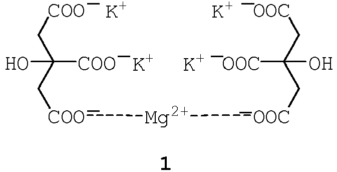 and optionally,
(iv) one or more auxiliary food ingredients,
wherein the said ingredients are used in the following mass fractions:
(i) sodium chloride, 64-65% w/w;
(ii) potassium chloride, 24-25% w/w;
(iii) potassium magnesium citrate (**1**), 8-12% w/w; and optionally,
(iv) one or more auxiliary food ingredients, 0-3% w/w.

The above salt substitute may be used as a blood pressure lowering agent.

Sodium chloride (NaCl) that can be used in the composition of this invention includes all commercially available food grade common salts from either salt mines or sea salt. The suitable product involves all available fine crystalline forms, most preferably of particles size from 0.1-0.6 mm.

Potassium chloride (KCl) that can be employed in the composition involves all available food grade products in fine crystalline forms, most suitable of particles size from 0.1-0.6 mm.

Sodium chloride and potassium chloride as starting raw materials can be those processed with adequate amounts of food grade anti-caking agents such as sodium ferrocyanide and/or magnesium carbonate or can be used without any anti-caking agent.

Potassium magnesium citrate (**1**) used in this invention is of specific chemical composition represented by the chemical formula K₄Mg(C₆H₅O₇)₂, with stoichiometric ratio of:
**n(K) : n(Mg) : n(citrate)** = **4 : 1 : 2.**

The salt 1 was used as commercially available food grade, white, fine powderous product.

The auxiliary food ingredient is selected from the group comprising: maltodextrin; dextrin; starch; cereal flours; mannitol; edible fats and oils; anti-caking agent; or mixtures of these substances.

Edible fats and oils include all edible triglycerides such as sunflower oil, soybean oil, coconut oil, palm oil, hydrogenated triglycerides, other edible fats and plant oils, or mixtures of these ingredients.

Anti-caking agent is used in powderous salt substitute form as an additive which prevents lumping of the formulation. The latter is selected from the group comprising: silicon dioxide; magnesium carbonate; stearic acid; magnesium stearate; calcium silicate; sodium ferrocyanide; sodium aluminium silicate; magnesium silicates; calcium silicate; talc; other food grade anti-caking agents; or mixtures of these substances.

### Preparation of the composition from the present invention

The composition of the present invention is typically in fine powderous mixture suitable for salting foods. Such product is obtained by:
1. mixing (dry homogenization) of fine crystalline and/or powderous ingredients (i), (ii), (iii), and optionally (iv) furnishing fine homogeneous crystalline powder mixture; or
2. by dissolution of ingredients (i), (ii), (iii), and optionally (iv) in suitable amount of food grade purified water to yield concentrated aqueous solution, which is subsequently spray-dried into small granules of practically useful particles size, e.g. 0.1-1 mm.

Both processes can be performed in standard industrial equipment that are usually employed in food and chemical industries. The product can be packed:
(i) into plastic containers or various bags in powder or granules form for use as salt substitute, seasoning, or industrial salt substitute; or
(ii) in small bags with divided dosages for use as dietetic salt or pharmaceutical adjuvant for treatment of diseases or conditions in humans and animals.

The composition of the present invention is typically produced with -35% of salt (NaCl) content relatively to the same amount (weight) of ordinary salt (NaCl). Representative experimental preparations are described in Examples 1-6. Of these, example 3 illustrates compositions according to the invention.

### Sensory study of the salt substitute composition from the present invention

We have found that the composition of the present invention acts as very efficient salt substitute with the following sensory characteristics:
(i) practically clean salty taste;
(ii) a good level of saltiness intensity; and
(iii) practically without any strange (foreign) taste.

The methodology of the sensory testing was similar to those described in literature references 11 and 12:
11) EP1776875A1; M. Kuroda, Y. Nozawa: Seasoning composition, seasoning material and process for producing food therewith; and
12) EP2446752A1; S. Chiba: Composition for low-salt food or beverage; both of Ajinomoto Co. Inc., Japan.

The testing was performed by a six-membered panel which evaluated the following parameters or tastes: (1) saltiness; (2) metallic; (3) bitterness; (4) sweetness; and (5) sourness.

The parameters were quantitatively scored from 0 to ++++, with allowed intermediary marks TR= traces, +/++, ++/+++, and +++/++++; Table 1.

**Table 1. Score criteria for sensory evaluation.**

| **No.** | **Taste intensity** | **Score** |
|---|---|---|
| 1 | no taste | 0 |
| 2 | taste in traces | TR |
| 3 | weak taste¹ | + |
| 4 | taste of medium intensity, | ++ |
| | clear effect of respective taste² | |
| 5 | strong taste, | +++ |
| | strong effect of respective taste³ | |
| 6 | very strong taste, | ++++ |
| | very strong effect of respective taste⁴ | |
| In the case of doubt, intermediary scores TR= traces, +/++, ++/+++, and +++/++++ were allowed. | | |

| | | |
|---|---|---|
| ¹ Equivalent saltiness intensity of 0.4-0.5% w/w of aqueous NaCl solution. ² Equivalent saltiness of 0.7-0.8% w/w of aqueous NaCl. ³ Equivalent saltiness of 1.0-1.2% w/w of aqueous NaCl. ⁴ Equivalent saltiness of 1.5-1.7% w/w of aqueous NaCl. | | |

An initial testings were performed in water at 1% w/w concentration of salt substitute at two relative concentrations of potassium chloride (KCl) versus sodium chloride (NaCl) (both compositions being comparative):
(i) 72% KCl + 18% NaCl + 10% taste-improving agent = 100% w/w (KCl:NaCl= 4:1 w/w); and
(ii) 60% KCl + 30% NaCl + 10% taste-improving agent = 100% w/w (KCl:NaCl= 2:1 w/w).

Taste-improving agents that were tested are:
(i) potassium magnesium citrate (**1**) ; versus
(ii) tripotassium citrate (K₃C₆H₅O₇), potassium dihydrogencitrate [KH₂(C₆H₅O₇)], trimagnesium citrate [Mg₃(C₆H₅O₇)₂], and magnesium hydrogencitrate [MgH(C₆H₅O₇)] that all can be considered as prior art solutions.

Experimental procedure for testing is described in Example 7. Results are presented in Table 2.

**Table 2. The sensory testing of salt substitutes at concentration of 1% w/w in water medium and relative ratio of KCl:NaCl= 4:1 w/w. Influence of various taste-improving agents: potassium magnesium citrate [1; K₄Mg(C₆H₅O₇)₂] versus various citrate salts from the prior art.^{a}**

| **No.** | **Taste-improving agent** | **Taste intensity** | | | | | **pH** |
|---|---|---|---|---|---|---|---|
| | | **S** | **M** | **B** | **SW** | **A** | |
| 1 | Standard (STD-1)^{b} | ++ | ++ | TR | 0 | 0 | 7.5-8.0 |

| **Prior art:** | | | | | | | |
|---|---|---|---|---|---|---|---|
| 2 | K₃C₆HₛO₇^{c} | ++ | + | 0 | + | 0 | 7.8-8.3 |
| 3 | KH₂ (C₆H₅O₇) | ++ | TR | 0 | 0 | + | 6.0-6.5 |
| 4 | Mg₃(C₆H₅O₇)₂^{e} | ++ | + | 0 | 0 | 0 | 7.7-8.3 |
| 5 | MgH(C₆H₅O₇)^{f} | ++ | + | 0 | 0 | TR | 7.0-7.5 |
| | | | | | | | |
| 6 | K₄Mg(C₆H₅O₇)₂^{g} | ++ | TR/+ | 0 | TR/+ | 0 | 7.5-8.0 |

| | | | | | | | |
|---|---|---|---|---|---|---|---|
| S= saltiness; M= metallic; B= bitterness; SW= sweetness; A= sourness, acidic ^{a} Testings were performed in water medium. Absolute concentrations were: 0.72% KCl, 0.18% NaCl, 0.10% taste-improving agent; total concentration was 1% w/w of each salt substitute tested. ^{b} Standard (STD-1) was 0.9% w/w aqueous solution of KCl:NaCl= 4:1, w/w; absolute concentrations: 0.72% KCl, 0.18% NaCl. ^{c} Significant improvement, less metallic but slightly increased sweetness. ^{d} Metallic taste in traces, but strong acidic influence. ^{e} Significant improvement, less metallic than STD-1, better than K₃C₆H₅O₇. ^{f} Significant improvement, less metallic than STD-1, similar to Mg₃(C₆H₅O)₇)₂, but generates acidic strange taste. ^{g} Significant improvement over Mg₃(C₆H₅O₇)₂, no sour taste as KH₂(C₆H₅O₇); very weak (TR/+) sweet taste, this was almost negligible. | | | | | | | |

The same testing was performed at relative ratio of KCl:NaCl= 2:1 w/w, (comparative) at the same concentration of 1% w/w of model salt substitute compositions. Results are given in Table 3.

**Table 3. The sensory testing of salt substitutes at concentration of 1% w/w in water medium and KCl:NaCl= 2:1 w/w. Influence of various taste-improving agents: potassium magnesium citrate [1; K₄Mg(C₆H₅O₇)₂] versus various citrate salts from the prior art.^{a}**

| **No.** | **Taste-improving agent** | **Taste intensity** | | | | | **pH** |
|---|---|---|---|---|---|---|---|
| | | **S** | **M** | **B** | **SW** | **A** | |
| 1 | standard (STD-2)^{b} | ++ | ++ | TR | 0 | 0 | 7.5-8.0 |

| **Prior art:** | | | | | | | |
|---|---|---|---|---|---|---|---|
| 2 | K₃C₆H₅O₇^{c} | ++ | + | 0 | + | 0 | 7.8-8.3 |
| 3 | KH₂(C₆H₅O₇)^{d} | ++ | TR | 0 | 0 | + | 6.0-6.5 |
| 4 | Mg₃(C₆H₅O₇)₂^{e} | ++ | + | 0 | 0 | 0 | 7.5-8.0 |
| 5 | MgH (C₆H₅O₇) ^{f} | ++ | + | 0 | 0 | TR | 7.0-7.5 |
| | | | | | | | |
| 6 | K₄Mg(C₆H₅O₇)₂^{g} | ++/+++ | TR | 0 | 0 | 0 | 7.5-8.0 |

| | | | | | | | |
|---|---|---|---|---|---|---|---|
| S= saltiness; M= metallic; B= bitterness; SW= sweetness; A= sourness, acidic ^{a} Testing was carried out in water medium. Absolute concentrations were: 0.60% KC1, 0.30% NaCl, 0.10% taste-improving agent; total concentration was 1% w/w of each salt substitute tested. ^{b} Standard (STD-2) was 0.9% w/w aqueous solution of KCl:NaCl= 2:1, w/w. ^{c} Significant improvement, less metallic but slightly increased sweetness. ^{d} Metallic taste in traces, but strong acidic influence. ^{e} Significant improvement, less metallic than STD-2, better than K₃C₆H₅O₇. ^{f} Significant improvement, less metallic than STD-2, similar to Mg₃(C₆H₅O7)₂, but generates acidic strange taste. ^{g} Significant improvement over Mg₃(C₆H₅O₇)₂, slightly more salty than STD-2, remained metallic off-taste was almost negligible (TR). | | | | | | | |

The results showed that all tested potassium and magnesium citrate salts do act as more or less effective taste improving agents in potassium chloride-based salt formulations, both at KCl:NaCl= 4:1 w/w and 2:1 w/w (both comparative).

Trimagnesium citrate [Mg₃(C₆H₅O₇)₂] was found to be relatively the most effective agent among prior art compounds at both KCl:NaCl w/w ratios.

Comparative analysis of trimagnesium citrate [Mg₃(C₆H₅O7)₂] versus potassium magnesium citrate [**1;** K₄Mg(C₆H₅O₇)₂] showed that the latter was equally effective in masking bitterness but:
(i) more effective in masking offensive metallic taste of KCl, practically without generation of any noticeable additional strange taste; and
(ii) slightly more salty than either STD-2 or trimagnesium citrate [Mg₃(C₆H₅O₇)₂].

Potassium dihydrogencitrate generates noticeable sour taste what can be also detected by the pH values of test solution which were significantly lower than in other cases.

Furthermore the similar testing was carried out with the same taste-improving agents at 1.5% w/w of the respective salt substitutes. Thus absolute concentrations of each component were as follow:
(i) 64% KCl + 16% NaCl + 20% taste-improving agent = 100% w/w;
(ii) 54% KCl + 26% NaCl + 20% taste-improving agent = 100% w/w.
(both comparative)

Results are given in Tables 4 and 5.

**Table 4. The sensory testing of salt substitutes at concentration of 1.5% w/w in water medium and KCl:NaCl= 4:1 w/w. Influence of various taste-improving agents: potassium magnesium citrate [1; K₄Mg(C₆H₅O₇)₂] versus various citrate salts from the prior art.^{a}**

| **No.** | **Taste-improving agent** | **Taste intensity** | | | | | **pH** |
|---|---|---|---|---|---|---|---|
| | | **S** | **M** | **B** | **SW** | **A** | |
| 1 | Standard (STD-3)^{b} | +++ | ++ | TR | 0 | 0 | 7.5-8.0 |

| **Prior art:** | | | | | | | |
|---|---|---|---|---|---|---|---|
| 2 | K₃C₆H₅O₇^{c} | +++ | TR/+ | 0 | TR | 0 | 7.5-8.0 |
| 3 | KH₂(C₆H₅O₇)^{d} | ++ | 0 | 0 | 0 | +++ | 4.4-5.0 |
| 4 | Mg₃(C₆H₅O₇)₂^{e} | +++ | TR/+ | 0 | + | 0 | 8.0-8.5 |
| 5 | MgH(C₆H₅O₇)^{f} | +++ | TR/+ | 0 | 0 | + | 6.4-6.7 |
| | | | | | | | |
| 6 | K₄Mg(C₆H₅O₇)₂^{g} | +++/++++ | TR | 0 | + | 0 | 8.0-8.5 |

| | | | | | | | |
|---|---|---|---|---|---|---|---|
| S= saltiness; M= metallic; B= bitterness; SW= sweetness; A= sourness, acidic ^{a} Testing was performed in water medium. Absolute concentrations were: 0.96% KCl, 0.24% NaCl, 0.30% taste-improving agent; total concentration was 1.5% w/w of each salt substitute tested. ^{b} Standard (STD-3) was 1.2% w/w aqueous solution of KCl:NaCl= 4:1, w/w; absolute concentrations: 0.96% KCl, 0.24% NaCl. ^{c} Significant improvement, less metallic but slightly increased sweetness. ^{d} Strong acidic taste, stronger than saltiness; not applicable as taste-improving agent at this concentration at all. ^{e} Significant improvement, less metallic than STD-3, comparable efficacy as K₃C₆H₅O₇. ^{f} Significant improvement, less metallic than STD-3, similar to Mg₃(C₆H₅O₇)₂, but generates significant acidic strange taste. ^{g} Significant improvement over Mg₃(C₆H₅O₇)₂; the strongest salty taste; minor (TR) residual metallic off-taste; no sour taste as KH₂(C₆H₅O₇); weak (+) sweet taste. | | | | | | | |

**Table 5. The sensory testing of salt substitutes (each comparative) at concentration of 1.5% w/w in water medium and KCl:NaCl= 2:1 w/w. Influence of various taste-improving agents: potassium magnesium citrate [1; K₄Mg(C₆H₅O₇)₂] versus various citrate salts from the prior art.^{a}**

| **No.** | **Taste-improving agent** | **Taste intensity** | | | | | **pH** |
|---|---|---|---|---|---|---|---|
| | | **S** | **M** | **B** | **SW** | **A** | |
| 1 | Standard (STD-4)^{b} | +++ | ++ | TR | 0 | 0 | 7.5-8.0 |

| **Prior art:** | | | | | | | |
|---|---|---|---|---|---|---|---|
| 2 | K₃C₆H₅O₇^{c} | +++ | TR | 0 | + | 0 | 7.5-8.0 |
| 3 | KH₂(C₆H₅O₇)^{d} | ++ | 0 | 0 | 0 | +++ | 4.4-5.0 |
| 4 | Mg3 (C₆H₅O₇)₂^{e} | +++ | + | 0 | 0 | 0 | 7.5-8.0 |
| 5 | MgH(C₆H₅O₇)^{f} | +++ | + | 0 | 0 | + | 6.4-6.7 |
| | | | | | | | |
| 6 | K₄Mg (C₆H₅O₇)₂^{g} | +++/++++ | TR | 0 | + | 0 | 8.0-8.5 |

| | | | | | | | |
|---|---|---|---|---|---|---|---|
| S= saltiness; M= metallic; B= bitterness; SW= sweetness; A= sourness, acidic ^{a} Testing was conducted in water medium. Absolute concentrations were: 0.80% KCl, 0.40% NaCl, 0.30% taste-improving agent; total concentration was 1.5% w/w of each salt substitute tested. ^{b} Standard (STD-4) was 1.2% w/w aqueous solution of KCl:NaCl= 2:1, w/w; absolute concentrations: 0.80% KCl, 0.40% NaCl. ^{c} Significant improvement, less metallic but slightly increased sweetness. ^{d} Strong acidic taste, stronger sour than salty; not applicable as taste-improving agent at this concentration at all. ^{e} Significant improvement, less metallic than STD-4, lower efficacy than K₃C₆H₅O₇. ^{f} Significant improvement, less metallic than STD-4, similar to Mg₃(C₆H₅O₇)₂, but generates additional acidic strange taste. ⁹ Significant improvement over K₃C₆H₅O₇ and expecially Mg₃ (C₆H₅O₇) ₂; the strongest salty taste; minor (TR) residual metallic off-taste; weak (+) sweet taste. | | | | | | | |

The results from these additional testing at higher concentrations (1.5% w/w) of salt substitutes with two KCl:NaCl ratios of 4:1 w/w and 2:1 w/w and 20% w/w of taste-improving agents showed that all tested potassium and magnesium citrate salts do act as more or less effective taste improving agents except potassium dihydrogencitrate [KH₂(C₆H₅O₇)] which generates to much acidity at this concentration. The latter was also detected by drastically lower pH value of 4.4-5.0.

The best prior art taste-improving agents are tripotassium citrate (K₃C₆H₅O₇) and then trimagnesium citrate [Mg₃(C₆H₅O₇)₂] as slightly less effective one.

Comparative analysis of potassium magnesium citrate [1; K₄Mg(C₆H₅O₇)₂] versus tripotassium citrate (K₃C₆H₅O₇) showed that the latter was similarly effective in masking bitterness, but more effective in covering offensive metallic taste of KC1, and with slightly stronger salty taste perception.

In addition, the composition of this invention was tested in a matrix of a model chicken vegetable soup, at 1.0% and 1.5% w/w concentrations, to test its performances against:
(i) the "golden standard", a common table salt (NaCl); and
(ii) a mixture of NaCl:KCl= 50:40 w/w which was considered as "negative control", see Table 6.

**Table 6. Results of sensory testing of the composition from example (described later) against the "golden standard", table salt (NaCl) and a mixture of NaCl:KCl= 50:40 w/w ("negative control").^{a}**

| **No.** | **Salt or salt substitute** | **Taste intensity** | | | | |
|---|---|---|---|---|---|---|
| | | **S** | **M** | **B** | **SW** | **A** |
| **1% w/w in the model soup** | | | | | | |
| 1 | NaCl | ++ | 0 | 0 | 0 | 0 |
| 2 | NaCl: KCl, 50:40^{b} | + | TR/+ | 0 | 0 | 0 |
| 3 | Example 2 | +/++ | 0 | 0 | 0 | 0 |

| **1.5% w/w in the model soup** | | | | | | |
|---|---|---|---|---|---|---|
| 4 | NaCl | ++++ | 0 | 0 | 0 | 0 |
| 5 | NaCl:KCl, 50:40^{b} | +++ | + | TR/+ | 0 | 0 |
| 6 | Example 2 | +++/++++ | 0 | 0 | 0 | 0 |

| | | | | | | |
|---|---|---|---|---|---|---|
| S= saltiness; M= metallic; B= bitterness; SW= sweetness; A= sourness, acidic ^{a} Sensory testing was performed at 1% and 1.5% w/w of taste-improving agent in model chicken vegetable soup served at 35-45 °C. ^{b} Mixture of NaCl:KCl, 50:40 w/w was used at concentrations of 90% from 1% and 1.5%, precisely 0.9% and 1.13% to the chicken soup, in order to check the contribution of K₄Mg(C₆H₅O₇)₂ in the formulation from Example 2 to the increasing of saltiness to the same basic mixture of NaCl:KCl, 50:40 w/w. | | | | | | |

These results showed that the composition of example 2 does act as effective salt (NaCl) substitute by:
(i) effective masking of bad off-flavours connected with KC1: metallic taste and bitterness;
(ii) slight increasing of salty taste perception in comparison with basic mixture of NaCl + KCl;
(iii) still sufficient level of overall saltiness; the saltiness of the same amount of the composition from the present invention is significantly lower than that of pure salt (NaCl), but in this matrix quite sufficient for a good saltiness level perception.

The preparation of model chicken vegetable soup, and sensory testing of the composition against table salt (NaCl) is described in Example 8.

Quantitative testing of saltiness of the composition from this invention (Example 3) which contained 65% NaCl, at 1.0% concentration in water at 20-25 °C, showed 81% of relative saltiness (average of 0.80-0.825%) against 72.5% (average of 0.70-0.75%) for a mixture of NaCl+KCl (65+35% w/w) and the "golden standard (NaCl; 100%)".

This represents -19% relative saltiness against common salt (NaCl) and +8% increasing above the relative saltiness of the similar mixture of NaCl+KCl (65+35%; "negative control") at concentration range of 1% w/w in aqueous solution at 20-25 °C, of course, with drastically improved taste of thus prepared solution.

### The use of the composition of taste or flavour enhancer from the invention

The composition of the present invention acts as effective salt substitute providing significantly reduced sodium intake, e.g. -30%, with excellent taste profile and minimally reduced salty taste perception, e.g. -10% to -19%.

The composition can be used as:
(i) seasoning agent;
(ii) table-top salt substitute;
(iii) an industrial salt replacement for manufacturing of various food products with reduced sodium content;
(iv) a food supplement for supplementation of human diet with sodium, potassium, and magnesium;
(v) a dietetic salt for prevention and treatment of diseases and conditions selected from the group consisting of: hypokalemia, hypomagnesemia, hypertension, diarrhea, hypokalemic acidosis, renal tubular acidosis, metabolic acidosis, Cushing's syndrome; and especially essential hypertension;
(vi) adjuvant for prevention and treatment of diseases and conditions selected from the group consisting of: arrhythmia, tachycardia, acute myocardial infarction, pre-eclampsia, asthma, acute pancreatitis, hemorrhagic shock, Crohn's disease, ulcerative colitis, inflammatory bowel diseases, alcoholism, and chronic stress;
(vii) salt substitute in animal nutrition;
(viii) salt replacement for manufacturing of animal feed products;
(ix) an animal feed supplement for supplementation animal diet with sodium, potassium, and magnesium; and
(x) dietetic salt for prevention and treatment of animal diseases wherein supplementation with potassium and magnesium provides relief thereof.

As stated above, the composition of this salt substitute can be employed as industrial salt replacement in manufacturing of various food products with reduced sodium content. Such food products are selected from the group comprising:
(i) culinary products such as soups, bouillons, food seasonings, and sauces;
(ii) dry foods including snacks, cereals, and biscuits;
(iii) meat products including ham, sausages, and pates;
(iv) milk products such as processed milk or fermented milk products;
(v) bakery products including bread and baked confections;
(vi) fats and processed oily foods like salad oils, margarine, butter, mayonnaise, salad dressings, and shortenings;
(vii) marine products;
(viii) prepared meals like frozen, sterilized, or chilled products and foodservice products;
(ix) nutritional products including foods for special nutritional purposes;
(x) flavours and flavour ingredients;
(xi) beverages;
(xii) animal feed; or
(xiii) any other food product wherein salt or other salty-taste ingredient is part of the composition.

The recommended dosage of the composition is from 0.01-25% w/w of the respective food product.

### Examples

### General remarks

All quantitative compositions are given in mass fractions, meaning weight percentages (% w/w).

Sodium chloride was used in the form of fine food grade salt of type Gustosal, purchased from Salinen Austria AG, Austria. Potassium chloride was employed in the form of fine food grade crystalline product of type KaliSel, purchased from K+S KALI GmbH, Germany.

Potassium magnesium citrate (1) was in the form of white fine powder, purchased from Dr. Paul Lohmann GmbH KG, Germany.

Room temperature (r.t.) means the temperature interval of 20-25 °C.

### Example 1 (comparative): Preparation of a composition with -70% sodium chloride

Composition (for 100 g of crystalline powder):
(1) 30.00 g (30.00%) sodium chloride
(2) 49.00 g (49.00%) potassium chloride
(3) 20.00 g (20.00%) potassium magnesium citrate (1)
(4) 1.00 g (1.00%) colloidal silicon dioxide, type Aerosil 200F¹

Preparation: The ingredients (1-4) were placed into the V-blender and homogenized by mixing for 15 minutes.

The product was in the form of white fine crystalline, flowable powder; composition: 11.8% Na, 31.2% K, 0.87% Mg.

### ¹ Product of Evonik Industries AG, Germany.

### Example 2 (Comparative). Preparation of a composition with -50% sodium chloride

Composition (for 100 g of crystalline powder):
(1) 50.00 g (50.00%) sodium chloride
(2) 37.25 g (37.25 %) potassium chloride
(3) 12.00 g (12.00%) potassium magnesium citrate (**1**)
(4) 0.75 g (0.75%) magnesium carbonate, light, food grade¹

Preparation: The ingredients (1-3) were placed into the V-blender and homogenized by mixing for 15 minutes. Then magnesium carbonate was added, and further homogenized for 5 minutes. The product was in the form of white fine crystalline, flowable powder; composition: 19.6% Na, 22.8% K, 0.52% Mg.

### ¹ Product of Dr. Paul Lohmann GmbH KG, Germany.

### Example 3. Preparation of the composition of the invention with -35% sodium chloride

Composition (for 100 g of crystalline powder):
(1) 65.00 g (65.00%) sodium chloride
(2) 24.00 g (24.00 %) potassium chloride
(3) 10.00 g (10.00%) potassium magnesium citrate (**1**)
(4) 1.00 g (1.00%) magnesium carbonate, light, food grade¹

Preparation: The ingredients (1-3) were placed into the V-blender and homogenized by mixing for 15 minutes. Then (4) was added, and further homogenized for 5 minutes.

The product was in the form of white fine crystalline, flowable powder; composition: 25.5% Na, 15.3% K, 0.43% Mg.

### ¹ Product of Dr. Paul Lohmann GmbH KG, Germany.

### Example 4 (comparative): Preparation of a composition with -25% sodium chloride

Composition (for 100 g of crystalline powder):
(1) 75.00 g (75.00%) sodium chloride
(2) 16.00 g (16.00 %) potassium chloride
(3) 8.00 g (8.00%) potassium magnesium citrate (**1**)
(4) 1.00 g (1.00%) colloidal silicon dioxide, type Aerosil 200F¹

Preparation: The ingredients (1-3) were placed into the V-blender and homogenized by mixing for 15 minutes. Then (4) was added, and further homogenized for 5 minutes.

The product was in the form of white fine crystalline, flowable powder; composition: 29.4% Na, 10.6% K, 0.34% Mg.

### ¹ Product of Evonik Industries AG, Germany.

### Example 5 (comparative): Preparation of a composition with -20% sodium chloride

Composition (for 100 g of crystalline powder):
(1) 80.00 g (80.00%) sodium chloride
(2) 14.00 g (14.00 %) potassium chloride
(3) 5.00 g (5.00%) potassium magnesium citrate (1)
(4) 1.00 g (1.00%) colloidal silicon dioxide, type Aerosil 200F¹

Preparation: The ingredients (1-3) were placed into the V-blender and homogenized by mixing for 15 minutes. Then (4) was added, and further homogenized for 5 minutes.

The product was in the form of white fine crystalline, flowable powder; composition: 31.4% Na, 8.7% K, 0.22% Mg.

### Example 6 (comparative): Preparation of a composition with -50% sodium chloride by spray-drying

Composition (for 100 g of microgranulated product):
(1) 50.00 g (50.00%) sodium chloride
(2) 37.00 g (37.00 %) potassium chloride
(3) 12.00 g (12.00%) potassium magnesium citrate (**1**)
(4) 1.00 g (1.00%) magnesium carbonate, light, food grade¹
(5) q.s. food grade purified water

Preparation: The ingredients (1-3) were dissolved in 300 ml of food grade purified water by stirring in 500 ml baker with magnetic stirrer at room temperature during 15 minutes. Then (4) was added and suspended in thus obtained solution by stirring for 5 minutes.

Thus obtained solution/suspension was spray dried.

The product was in the form of white fine granules; composition: 19.6% Na, 22.7% K, 0.52% Mg. ¹ Product of Dr. Paul Lohmann GmbH KG, Germany.

### Example 7. Sensory evaluation of the composition from the present invention. Efficacy of potassium magnesium citrate (1) as taste-improving agent versus various citrate salt from the prior art.

The methodology of the sensory testing was similar to those described in literature references 11 and 12. The testing was performed by a six-membered panel which evaluated the following parameters or tastes: (1) saltiness; (2) metallic; (3) bitterness; (4) sweetness; and (5) sourness. The parameters were quantitatively scored from 0 to ++++, with allowed intermediary marks TR= traces, +/++, ++/+++, and +++/++++; see Table 1. In the case of doubt, intermediary scores TR= traces, +/++, ++/+++, and +++/++++ were allowed.

The following taste-improving agents in model salt substitutes were tested: 1. tripotassium citrate (K₃C₆H₅O₇), 2. potassium dihydrogencitrate [KH₂(C₆H₅O₇)], 3. trimagnesium citrate [Mg₃(C₆H₅O₇)₂], and 4. magnesium hidrogencitrate [MgH(C₆H₅O₇)] from the prior art, as well as 5. potassium magnesium citrate [K₄Mg(C₆H₅O₇)₂] which is used in this invention.

### Preparation of model salt substitutes. General procedure:

A) Model salt substitutes with KCl:NaCl= 4:1 w/w, and 10% taste-improving agent (100 g scale): 72.00 g of potassium chloride, 18.00 g sodium chloride, and 10.00 g taste-improving agent was placed into V-blender and homogenized by mixing for 20 minutes. Thus obtained products were used as salt substitutes for the sensory study whose results are given in Table 2.
B) Model salt substitutes with KCl:NaCl= 2:1 w/w, and 10% taste-improving agent (100 g scale): 60.00 g of potassium chloride, 30.00 g sodium chloride, and 10.00 g taste-improving agent was placed into V-blender and homogenized by mixing for 20 minutes. Thus obtained products were used as salt substitutes for the sensory study whose results are given in Table 3.
C) Model salt substitutes with KCl:NaCl= 4:1 w/w, and 20% taste-improving agent (100 g scale): 64.00 g of potassium chloride, 16.00 g sodium chloride, and 20.00 g taste-improving agent was placed into V-blender and homogenized by mixing for 20 minutes. Thus obtained products were used as salt substitutes for the sensory study whose results are given in Table 4.
D) Model salt substitutes with KCl:NaCl= 2:1 w/w, and 20% taste-improving agent (100 g scale): 54.00 g of potassium chloride, 26.00 g sodium chloride, and 20.00 g taste-improving agent was placed into V-blender and homogenized by mixing for 20 minutes. Thus obtained products were used as salt substitutes for the sensory study whose results are given in Table 5.

An initial testing was performed in water at 1% w/w concentration of model salt substitutes described under A) and B). Results are presented in Tables 2 and 3.

Furthermore the similar testing was carried out with the same taste-improving agents at 1.5% w/w of the respective salt substitutes C) and D). Results are given in Tables 4 and 5.

The results from testing at both concentrations (1% and 1.5% w/w) of salt substitutes with two KCl:NaCl ratios of 4:1 and 2:1 w/w, and 10% and 20% w/w of taste-improving agents, showed that all tested potassium and magnesium citrate salts do act as more or less effective taste improving agents except potassium dihydrogencitrate [KH₂(C₆H₅O₇)] which, at higher concentration (1.5% w/w) generates to much acidity and is not applicable.

Trimagnesium citrate [Mg₃(C₆H₅O₇)₂] was found to be the most effective agent among prior art compounds at both KCl:NaCl ratios at lower (1% w/w) concentration, whilst tripotassium citrate (K₃C₆H₅O₇) was slightly more effective at higher (1.5% w/w) concentration.

Potassium magnesium citrate [1; K₄Mg(C₆H₅O₇)₂] was significantly better than either trimagnesium citrate [Mg₃(C₆H₅O₇)₂] or tripotassium citrate (K₃C₆H₅O₇) providing similar bitterness masking action but better metallic taste elimination and slightly higher salty taste perception.

### Example 8. Sensory evaluation of the composition from the present invention. Comparison of the composition from example 2 versus common table salt (NaCl).

The methodology of the sensory testing is explained in Example 7. The composition of this invention was tested in a matrix of a model chicken and vegetable soup, at 1.0% and 1.5% w/w concentrations, to test is performances against the "golden standard", common table salt (NaCl) and a mixture of NaCl:KCl = 50:40 w/w as "negative control". The latter mixture of NaCl:KCl, 50:40 was used at concentrations of 90% from 1% and 1.5%, precisely 0.9% and 1.13%, in order to check the contribution of K₄Mg(C₆H₅O₇)₂ in the formulation from Example 2 to the increasing of saltiness to the same basic mixture of NaCl:KCl, 50:40 w/w.

Preparation of the model chicken-vegetable soup: this was prepared from chicken wings (150 g) and sliced vegetables: onion (50 g), celery root (50 g), and carrot (100 g). The mixture was cooked in mildly boiling water (1000 ml) for 1 h. Then, the soup was allowed to cool to 50-60 °C, and decanted to remove all meat and vegetables. Total volume of thus obtained decantate was adjusted to 1000 ml with addition of warm pot water. Thus obtained product was used as a model soup for sensory testing. All samples of chicken meat and vegetables are used from the same batch to avoid their variability.

Preparation of the test soups: to the model soup (100 ml), salt or salt substitute:
(i) the "golden standard", NaCl (1.00 g or 1.50 g); or
(ii) composition of this invention from Example 2 (1.00 g or 1.50 g) ;
(iii) or the "negative control", a mixture of NaCl (0.50 g or 0.75 g) + KCl (0.40 g or 0.60 g) was added.
The mixture was stirred for 5 minutes and served at 35-45 °C. The results are described in Table 6.

### Industrial Applicability

The composition from the present invention is used for manufacturing of salt substitute that is employed as: sole seasoning agent; table-top salt replacement; salt substitute for manufacturing of various food products and animal feed products; as food or animal feed supplement for supplementation of human or animal diet with sodium, potassium, and magnesium; as adjuvant for prevention and treatment of diseases connected with lower nutritional intake of potassium and magnesium. Therefore, industrial applicability of this invention is obvious.

## Claims

1. A salt substitute composition consisting of:
(i) sodium chloride,
(ii) potassium chloride,
(iii) potassium magnesium citrate <K₄Mg(C₆H₅O₇)₂; **1**> 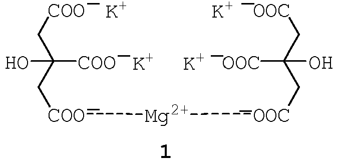 ana
(iv) one or more auxiliary food ingredients,
**characterized by that** the said ingredients are used in the following mass fractions:
(i) sodium chloride, 64-65% w/w,
(ii) potassium chloride, 24-25% w/w,
(iii) potassium magnesium citrate (1), 8-12% w/w, and
(iv) auxiliary food ingredients, 0-3% w/w.

2. A salt substitute composition according to the claim 1, wherein the auxiliary food ingredients (iv) are selected from the group consisting of: maltodextrin, dextrin, starch, cereal flours, mannitol, edible fats and oils, anti-caking agent or mixtures of these ingredients.

3. A process for the preparation of a salt substitute composition according to any of claims 1-2, wherein it involves homogenization of ingredients (i), (ii), (iii), and optionally ingredients (iv).

4. Use of the composition according to any of claims 1-2 as a seasoning agent, as a table-top salt substitute or as an industrial salt replacement for manufacturing of food products with reduced sodium content.

5. Use according to the claim 4, wherein the food product with reduced sodium content is taken from the group consisting of: culinary products such as soups, bouillons, food seasonings, and sauces; dry foods including snacks, cereals, and biscuits; meat products including ham, sausages, and pates; milk products such as processed milk or fermented milk products; bakery products including bread and baked confections; fats and processed oily foods like salad oils, margarine, butter, mayonnaise, salad dressings, and shortenings; marine products; prepared meals like frozen, sterilized, or chilled products and foodservice products; nutritional products including foods for special nutritional purposes; flavours and flavour ingredients; beverages; animal feed; or any other food product wherein salt or other salty-taste ingredient is a part of the composition.

6. Use according to any of the claims 4 or 5, wherein the composition is used in the range 0.01-25% w/w of the food product.

7. Composition according to any of claims 1-2, which is suitable for use as a food supplement for supplementation of human diet with sodium, potassium and magnesium.

8. Composition according to any of claims 1-2 for use as a dietetic salt in a method for the prevention and treatment of diseases and conditions selected from the group consisting of: hypokalemia, hypomagnesemia, hypertension, diarrhea, hypokalemic acidosis, renal tubular acidosis, metabolic acidosis, and Cushing's syndrome.

9. Composition for use according to claim 8, wherein the hypertension is essential hypertension.

10. Composition according to any of claims 1-2 for use as an adjuvant in a method for the prevention and treatment of diseases and conditions selected from the group consisting of: arrhythmia, tachycardia, acute myocardial infarction, pre-eclampsia, asthma, acute pancreatitis, haemorrhagic shock, Crohn's disease, ulcerative colitis, inflammatory bowel diseases, alcoholism, and chronic stress.

## Patentansprüche

1. Die Zubereitung, die als Salzersatz dient, die Folgendes enthält:
(i) Natriumchlorid,
(ii) Kaliumchlorid,
(iii) Kalium-Magnesiumcitrat <K₄Mg(C₆H₅O₇)₂; 1> 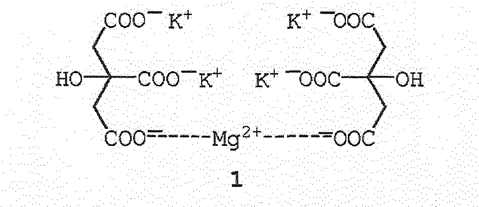 und
(iv) eine oder mehrere ernährende Hilfszutaten,
**dadurch gekennzeichnet, dass** die angeführten Zutaten in folgenden Massenanteilen gebraucht werden:
(i) Natriumchlorid, 64-65 % m/m,
(ii) Kaliumchlorid, 24-25 % m/m,
(iii) Kalium-Magnesiumcitrat (1),8-12 % m/m und
(iv) ernährende Hilfszutaten, 0-3 % m/m.

2. Zubereitung, die als Salzersatz dient, nach Anspruch 1, **dadurch gekennzeichnet, dass** die ernährenden Hilfszutaten (iv) ausgewählt sind aus der Gruppe bestehend aus: Maltodextrin, Dextrin, Stärke, Getreidemehl, Mannit, Speisefetten und -ölen, Antibackmitteln oder aus der Mischung der oben genannten Zutaten.

3. Verfahren zur Herstellung der Zubereitung zur Verwendung als Salzersatz nach einem der Ansprüche 1 oder 2, **dadurch gekennzeichnet, dass** es die Homogenisierung der Zutaten (i), (ii), (iii), und optional der Zutaten (iv) umfasst.

4. Verwendung der Zubereitung nach einem der Ansprüche 1 oder 2 als Gewürzmittel, als Ersatz für Speisesalz oder als industrieller Salzersatz zur Herstellung von Lebensmitteln mit reduziertem Natriumgehalt.

5. Verwendung nach Anspruch 4, **dadurch gekennzeichnet, dass** das Lebensmittelprodukt aus der Gruppe genommen wird, zu der folgende Produkte gehören: kulinarische Produkte wie Suppen, Brühen, Gewürze für Nahrungsmittel und Saucen; getrocknete Lebensmittel, einschließlich Snacks, Getreideprodukte und Kekse; Fleischprodukte, einschließlich Schinken, Wurst und Pasteten; Milchprodukte wie verarbeitete Milch oder fermentierte Milchprodukte; Backwaren, einschließlich Brot und Süßspeisen; Fette und verarbeitete Lebensmittel auf der Basis von Ölen wie Salatölen, Margarine, Butter, Mayonnaise, Salatsaucen und Fetten; Meeresprodukte; Fertiggerichte wie tiefgefrorene, sterilisierte oder gekühlte Produkte und Lebensmittel; Lebensmittelprodukte, einschließlich Lebensmittel für bestimmte Ernährungszwecke; Aromen und Zutaten für Aromen; Getränke; Tierfutter oder jedes andere Lebensmittelprodukt, in dem Salz oder eine andere salzige Zutat ein Teil dieser Zubereitung ist.

6. Verwendung nach einem der Ansprüche 4 oder 5, **dadurch gekennzeichnet, dass** die Zubereitung im Bereich von 0,01 bis 25 % m/m des Lebensmittelprodukts verwendet wird.

7. Zubereitung nach einem der Ansprüche 1 oder 2, **dadurch gekennzeichnet, dass** sie zur Verwendung als Nahrungsergänzungsmittel für die Ergänzung der menschlichen Ernährung mit Natrium, Kalium und Magnesium geeignet ist.

8. Zubereitung nach einem der Ansprüche 1 oder 2, **dadurch gekennzeichnet, dass** sie zur Verwendung als Diätsalz gebraucht wird, in dem Verfahren zur Vorbeugung und Behandlung von Erkrankungen und Zuständen, ausgewählt aus der Gruppe, zu der folgende Erkrankungen und Zustände gehören: Hypokaliämie, Hypomagnesiämie, Hypertonie, Durchfall, hypokaliämische Azidose, renale tubuläre Azidose, metabolische Azidose und Cushing-Syndrom.

9. Zubereitung zur Verwendung nach Anspruch 8, **dadurch gekennzeichnet, dass** die Hypertonie eine essentielle Hypertonie ist.

10. Zubereitung nach einem der Ansprüche 1 oder 2, **dadurch gekennzeichnet, dass** sie als Hilfsmittel bei dem Verfahren zur Vorbeugung und Behandlung von Erkrankungen und Zuständen gebraucht wird, ausgewählt aus der Gruppe, zu der folgende Erkrankungen und Zustände gehören: Arrhythmie, Tachykardie, akuter Myokardinfarkt, Präeklampsie, Asthma, akute Pankreatitis, hämorrhagischer Schock, Morbus Crohn, Colitis ulcerosa, entzündliche Darmerkrankungen, Alkoholismus und chronischer Stress.

## Revendications

1. Composition de substitut de sel comprenant du:
(i) chlorure de sodium,
(ii) chlorure de potassium
(iii) citrate de potassium et de magnésium < K₄Mg(C₆H₅O₇)₂; **1**> 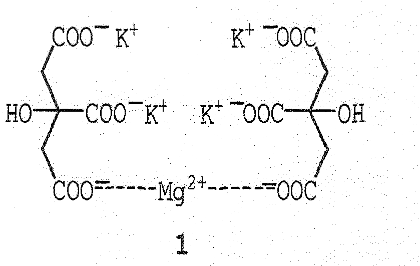 et
(iv) un ou plusieurs auxiliaires, **caractérisé en ce que,** ces derniers sont utilisés dans les fractions de masse suivante:
(i) chlorure de sodium, 64-65% w/w,
(ii) chlorure de potassium, 24-25% w/w,
(iii) citrate de potassium et de magnésium (1), 8-12% w/w, et
(iv) auxiliaires, 0-3% w/w.

2. Composition de substitut de sel selon la revendication 1, dans laquelle les auxiliaires sont choisis dans le groupe constitué par: la maltodextrine; la dextrine; l'amidon; les farines de céréales; le mannitol; les graisses et huiles comestibles; un anti-agglomérant ou des mélanges de ces ingrédients.

3. Procédé de préparation d'une composition de substitut de sel selon l'une quelconque des revendications 1 à 2, impliquant l'homogénéisation des ingrédients (i), (ii), (iii) et éventuellement des ingrédients (iv).

4. Utilisation de la composition selon l'une quelconque des revendications 1 à 2 comme agent d'assaisonnement, comme substitut de sel de table ou comme substitut de sel industriel pour la fabrication de produits alimentaires à teneur réduite en sodium.

5. Utilisation d'une composition selon la revendication 4, dans laquelle le produit alimentaire à teneur réduite en sodium est choisi dans le groupe comprenant: les produits culinaires tels que les soupes, les bouillons, les assaisonnements alimentaires et les sauces; les aliments secs, y compris les collations, les céréales et les biscuits; les produits à base de viande, notamment le jambon, les saucisses et les pâtés; les produits laitiers tels que le lait transformé ou les produits laitiers fermentés; produits de boulangerie, y compris pain et confiseries cuites au four; les graisses et les aliments gras transformés comme les huiles à salade, la margarine, le beurre, la mayonnaise, les vinaigrettes et les shortenings; produits marins; plats préparés comme les produits surgelés, stérilisés ou réfrigérés et les produits de restauration; produits nutritionnels, y compris aliments à des fins nutritionnelles spécifiques; arômes et ingrédients aromatiques; boissons; l'alimentation animale; ou tout autre produit alimentaire dans lequel du sel ou un autre ingrédient au goût salé fait partie de la composition.

6. Utilisation d'une composition selon l'une quelconque des revendications 4 ou 5, dans laquelle la composition est utilisée dans la fourchette de 0,01 à 25% w/w du produit alimentaire.

7. Composition selon l'une quelconque des revendications 1 à 2, destinée à être utilisée comme complément alimentaire pour la supplémentation de l'alimentation humaine avec du sodium, du potassium et du magnésium.

8. Composition selon l'une quelconque des revendications 1 à 2, destinée à être utilisée comme sel diététique pour la prévention et le traitement de maladies et d'affections sélectionnées parmi le groupe constitué par: l'hypokaliémie, l'hypomagnésémie, l'hypertension, la diarrhée, l'acidose hypokaliémique, l'acidose tubulaire rénale, l'acidose métabolique, et le syndrome de Cushing.

9. Composition pour une utilisation selon la revendication 8, dans laquelle l'hypertension est une hypertension essentielle.

10. Composition selon l'une quelconque des revendications 1 à 2, utilisée comme adjuvant dans un procédé pour la prévention et le traitement de maladies et affections sélectionnées parmi le groupe constitué par: l'arythmie, la tachycardie, l'infarctus aigu du myocarde, la pré-éclampsie, l'asthme, la pancréatite aiguë, le choc hémorragique, la maladie de Crohn, la colite ulcéreuse, les maladies inflammatoires de l'intestin, l'alcoolisme et le stress chronique.
